# EUROPEAN PATENT APPLICATION

(11) **EP 4 455 259 A1**
(43) Date of publication of application: **30.10.2024**
(21) Application number: 21968684.7
(22) Date of filing: 24.12.2021
(51) Int. Cl.: C12M 1/34, C12Q 1/68, C40B 20/02

(54) **DNA LIBRARY SEQUENCE FOR INCREASING NUMBER OF CHIP PROBES AND APPLICATION THEREOF**

(71) Applicant: BGI Shenzhen, Shenzhen, Guangdong 518083 (CN)
(72) Inventor: LIAO, Sha, Shenzhen, Guangdong 518083 (CN); CHEN, Ao, Shenzhen, Guangdong 518083 (CN); ZHANG, Wenwei, Shenzhen, Guangdong 518083 (CN); XU, Xun, Shenzhen, Guangdong 518083 (CN); CHEN, Xi, Shenzhen, Guangdong 518083 (CN); FU, Defeng, Shenzhen, Guangdong 518083 (CN)
(74) Representative: Dehns
(86) International application number: PCT/CN2021/141296
(87) International publication number: WO 2023/115555

(57) **Abstract**

A DNA library sequence for increasing the number of chip probes and an application thereof. The DNA library sequence comprises two or more position information labeling sequences and three or more fixed sequences, wherein the position information labeling sequences and the fixed sequences are arranged at intervals. The DNA library sequence increases the number of probes of a chip used for spatiotemporal omics, and then a capture number of transcript genes is increased. The present invention has a very high application prospect.

## Description

### TECHNICAL FIELD

The present disclosure belongs to the field of bioassays and specifically relates to a DNA library sequence for increasing the number of chip probes and an application thereof.

### BACKGROUND

Spatiotemporal omics correlates spatial information with molecular information through the combination of self-developed spatial array chips and genomics, and forms a bridge between genomics and clinical medicine by combining with the accumulated knowledge of clinical anatomy and imaging, thus opening up new breakthrough directions in the field of precision medicine. The capture chip used in spatiotemporal omics mainly utilizes the sequencing technology of the self-developed domestic second-generation high-throughput sequencing platform and open features to construct a sequence-specific library. After rolling circle amplification (RCA), DNA nanoballs (DNB) are formed and loaded onto the sequencing chip. The DNBs are sequenced and extended through hybriding oligonucleotide chains, and the DNBs are digested. Finally, the position of each DNB will obtain a complete probe sequence that has a unique position information label and can capture transcripts.

At present, the library used to make the spatiotemporal omics capture chip only sequences a segment of position information label for each copy after generating DNB. Theoretically, one copy can only generate one probe in the end. When the number of copies cannot be larger, it is difficult to increase the number of probes. However, the number of probes will affect the number of transcript genes ultimately captured. The increase in the number of probes will correspondingly promote the increase in the number of genes. Therefore, it is necessary to find a method to increase the number of probes on the capture chip.

### CONTENT OF THE PRESENT INVENTION

In response to the difficulty of increasing the number of probes using the existing library for making capture chips, the present disclosure provides a new method for constructing a library. By designing different libraries, each copy carries two or more segments of position information labels after generating DNB. Theoretically, each copy can eventually generate two or more probes, thereby increasing the number of probes on the capture chip.

The above technical solution allows each copy of the DNB produced using this library to generate multiple probe sequences. Compared to the original library where each copy only generates one probe sequence, this method has the advantages of increasing the number of probes and improving the capture number of transcript genes.

By increasing the number of position information labels in the library, the present disclosure obtains corresponding information for each segment of position information label during DNB sequencing after loading the chip. After the obtaining, the DNB is digested, and then a probe is generated based on each sequenced segment of position information label. Finally, each DNB copy can obtain multiple complete probe sequences that have unique position information labels and can capture transcripts. However, with the library using current position information label, each DNB copy can only generate one probe sequence.

The present disclosure mainly solves the above technical problems through the following technical solutions.

The present disclosure provides a DNA library sequence for increasing the number of chip probes, which comprises two or more position information labeling sequences and three or more fixed sequences, wherein the position information labeling sequences and the fixed sequences are arranged at intervals.

When the number of the position information labeling sequences is two, the DNA library sequence comprises, in order from a direction of 5' to 3': a first fixed sequence, a first position information labeling sequence, a second fixed sequence, a second position information labeling sequence, and a third fixed sequence.

The number of the position information labeling sequences may be 3, 4, or 5, or even more.

Optionally, a third position information labeling sequence and a fourth fixed sequence connected downstream to the third fixed sequence; and optionally up to an nth position information labeling sequence and an (n+1)th fixed sequence; n is an integer greater than 3.

The position information labeling sequence is composed of random bases (NNNNNN...), wherein each N is independently A, T, C, or G. Each random sequence is different. The length of the random sequence may range from 1 base to thousands of bases, and may be selected according to the number of position information labeling sequences, and is preferably between 5 bases and 50 bases.

The fixed sequence preferably contains a sequencing primer required for decoding the position information labeling sequence and a sequence that connects position information with cDNA of a subsequent single-cell transcriptome, and may be freely synthesized according to experimental purposes. The library sequence may be a single-stranded DNA or a doublestranded DNA.

In a preferred embodiment of the present disclosure, a first fixed sequence and a last fixed sequence of the DNA library sequence are each connected to an amplification site for molecular amplification. DNA libraries may be molecularly amplified before being loaded onto a solid surface, or after being loaded onto a solid surface, or both before and after being loaded. The amplification method may be bridging PCR amplification, rolling circle amplification (RCA), multiple displacement amplification (MDA), and emulsion PCR amplification.

In another preferred embodiment of the present disclosure, the DNA library sequence is connected to a nucleic acid molecule comprising a capture sequence capable of capturing nucleic acid in a sample.

The present disclosure also provides a kit, which comprises:
(1) the DNA library sequence as described above;
(2) a reaction mix for amplification.

The present disclosure also provides a method for preparing a chip with an increased number of probes, the method containing:
(1) synthesizing the DNA library sequence as described above;
(2) amplifying the DNA library sequence;
(3) decoding the position information labeling sequence by sequencing;
(4) adding a capture sequence.

Preferably, the sequencing in step (3) is ligation sequencing or sequencing by synthesis.

Preferably, the amplification is bridging PCR amplification, rolling circle amplification, multiple displacement amplification, or emulsion PCR amplification.

The present disclosure also provides a chip with an increased number of probes prepared by the method as described above.

The present disclosure also provides a use of the DNA library sequence as described above, the kit as described above, or the chip as described above in a method for detecting spatial information of a nucleic acid in a sample.

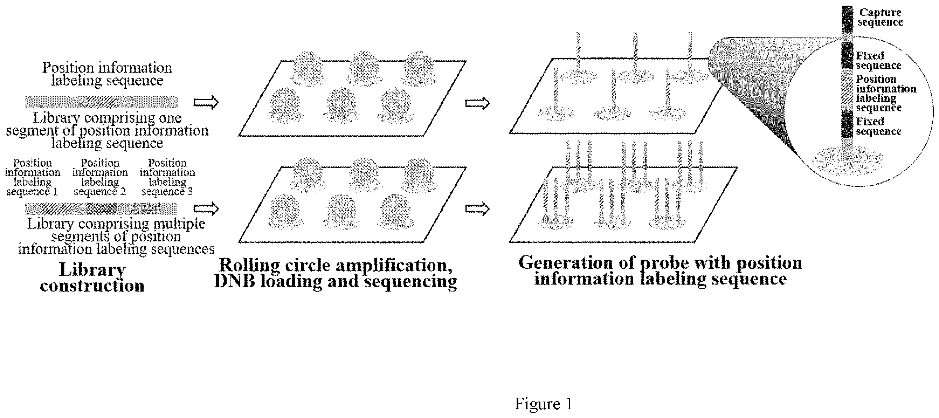

In the present disclosure, the method for obtaining the probe sequence can be a conventional method in the art, for example, it can be obtained through the following process (Figure 1):
(1) Synthesizing a DNA library sequence having a tissue localization region.
(2) Amplification of a position information labeling sequence. The synthesized DNA library with position information is loaded onto a solid surface, thereby each DNA molecule can label the position information of the solid surface.
(3) Decoding the position information through sequencing. According to the fixed sequence of the library described in (1), sequencing primers are designed, and spatially positioned random base sequences are decoded through sequencing, thereby the position information on the chip is labeled with DNA sequence information. The sequencing method may be ligation sequencing or sequencing by synthesis.
(4) Adding a capture sequence. In order to capture intracellular RNA information, sequences that capture cellular RNA molecules are ligated or synthesized on each DNA molecule having a position information sequence by ligation or polymerized. The capture sequence comprises a unique molecular identifier (UMI) for quantification of RNA molecules.

The positive progressive effects of the present disclosure are as follows:

With the help of the DNA library comprising multiple segments of position information labeling sequence and the method for preparing a chip with increased number of probes using the DNA library provided by the present disclosure, the number of probes on the chip used for spatiotemporal omics can be increased, thereby improving the capture number of transcript genes.

### BRIEF DESCRIPTION OF THE DRAWINGS

Figure 1 shows a flow chart for preparing a chip with an increased number of probes using the DNA library of the present disclosure.
Figure 2 shows probe signal detection results of the chip of the present disclosure.
Figure 3 shows a comparative diagram of probe signal detection values of chips prepared using the DNA library comprising multiple segments of position information labeling sequences of the present disclosure and a prior art DNA library comprising one segment of position information labeling sequence.

### DETAILED DESCRIPTION OF THE PREFERRED EMBODIMENT

### Example 1

1. A DNA library sequence having multiple segments of a molecular tag was designed and synthesized, and the base sequence was as shown below. Sequence synthesis was commissioned to BGI (Liuhe).
5'GAACGACATGGCGAGAT (first fixed sequence) NNNNNNNNNNNNNNNNNNNN (first position information labeling sequence, N represents any base, such as C, G, A, or T) GCCATGCCTCTCAGTACGTCAGCAGACCTATGCTGATAAGGTCTT (second fixed sequence) NNNNNNNNNNNNNNNNNNNN (second position information labeling sequence) GCCATGCCTCTCAGTACGTCAGCAGCCTTGGCTCACA (third fixed sequence) 3'
2. *In-situ* amplification of library, preparation of DNA nanoball (DNB): 40 µl of the following reaction mix was prepared, and 80 fmol of DNA library having position sequence information was added thereto.

| Ingredient | Volume (µl) | Final concentration |
|---|---|---|
| DNA library sequence having a tissue localization region | 80 fmol (X) | |
| 10× phi29 buffer (self-produced by BGI) | 4 | 1X |
| 10 µM of DNB primer sequence synthesized by BGI (Liuhe) | 4 | 1 µM |
| (GCCATGTCGTTCGGAACCGAGTGT) | | |
| Molecular grade water | 32-x | |

The above reaction mix was placed in a PCR instrument for reaction under the following conditions: 95°C for 3 minutes, then 40°C for 3 minutes. After the reaction was completed, the mix was placed on ice, and 40 µl of mixed enzyme I required for DNB preparation in DNBSEQ sequencing kit, 2 µl of mixed enzyme II, 1 µl of ATP (stock solution 100 mM, Thermo Fisher), and 0.1 µl of T4 ligase (self-produced by BGI) were added. After mixing, the above reaction mix was placed in a PCR instrument at 30°C for 20 minutes to form DNB. The DNB was loaded onto a BGISEQ500 sequencing chip according to the method described in BGISEQ500 SE50 kit.
3. Sequencing and decoding of position information labeling sequence. Sequencing was performed according to the instructions of the BGISEQ500 PE50 sequencing kit, with the SE set to a read length of 25 bp and the PE set to a read length of 25 bp. The MDA reagent in the BGISEQ500 PE50 sequencing kit was replaced with formamide. The fq file formed by sequencing was stored for later use.
4. MDA amplification. After the sequencing was completed, the excision reagent from the BGISEQ500 PE50 kit was pumped into the sequencing chip and incubated at 57°C for 5 minutes. Subsequently, a hybridization sequence ATGCCTCTCAGTACGTCAG was added, followed by the addition of the MDA reagent from the PE50 sequencing kit. After incubating at 37°C for 30 minutes, the chip was cleaned with 5×SSC.
5. The surface of the coupling chip was modified with NHS-N3 (Thermo Fisher), and after incubating for 30 minutes, the DBCO-modified sequence CTGCTGACGTACTGAGAGGCATGGC was pumped in and incubated overnight at room temperature.
6. 35 bp bases were polymerized. The polymerization reaction was carried out on the BGISEQ 500 platform according to the instructions of the BGISEQ500 PE50 sequencing kit, with the SE set to a read length of 35 bp. The photography option was deselected.
7. The DNB was digested, and 200 µl of the following S1 endonuclease (Thermo Fisher) mix was prepared, which was pumped into the sequencing chip and incubated at room temperature for 1 hour. Subsequently, residual fragments were removed by high-temperature denaturation.

| Ingredient | Volume (µl) |
|---|---|
| S1 | 1 |
| 5× reaction buffer | 40 |
| NucleaseFree water | 159 |

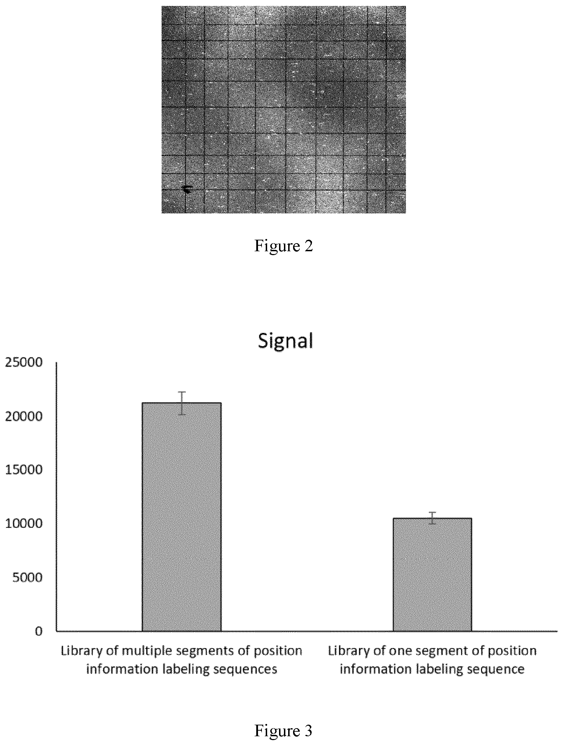

8. The sequences BAAAAAAAAAAAAAAAAAAAAAAAAANNNNNNNNNNACGACATGGCGAGAT and BAAAAAAAAAAAAAAAAAAAAAAAAANNNNNNNNNNTGCTGATAAGGTCTT synthesized by BGI (Liuhe) were hybridized. Subsequently, the MDA reagent from the BGISEQ500 PE50 kit was added and incubated at 30°C for 15 minutes.
9. For probe detection, high-temperature denaturation was used to convert the probe into a single-stranded structure. The cy5-modified sequence AAAAAAAAAAAAAAAAAAAAAAAAA was pumped in and incubated at room temperature for 10 minutes. Subsequently, photo detection was carried out on the BGISEQ500 (Figure 2). In the present disclosure, the complementary sequence of the probe was labeled with a dye, and after hybridization with the probe, the relative quantity of the probe was indicated by a fluorescent signal. In the present disclosure, ImageJ software was used to read the brightness of the photo, which was then converted into an average value as the detection value of the probe signal. A chip prepared from a prior art DNA library comprising one segment of position information labeling sequence was tested using the same method as described above. The results showed that when one segment of position information label was used in the prior art, the probe signal was 10500. With the improvement of multiple segments of position information labels, under the same detection conditions, the probe signal increased to 21200, indicating that the number of probes increased by nearly 100% (Figure 3). That is, both segments of position information labels generated probes. By increasing the number of position information labels, the yield of probes was improved. Under experimental conditions, the number of the segments of position information labels doubled, and the number of probes also doubled, which met the design expectations.

Although specific embodiments of the present disclosure have been described above, those skilled in the art should understand that these are only examples, and various changes or modifications can be made to these embodiments without departing from the principles and essence of the present disclosure. Therefore, the scope of protection of the present disclosure is defined by the appended claims.

## Claims

1. A DNA library sequence for increasing the number of chip probes, which comprises two or more position information labeling sequences and three or more fixed sequences, wherein the position information labeling sequences and the fixed sequences are arranged at intervals.

2. The DNA library sequence according to claim 1, wherein the DNA library sequence comprises, in order from a direction of 5' to 3': a first fixed sequence, a first position information labeling sequence, a second fixed sequence, a second position information labeling sequence, and a third fixed sequence;
optionally, a third position information sequence and a fourth fixed sequence connected downstream to the third fixed sequence; and optionally up to an nth position information labeling sequence and an (n+1)th fixed sequence; n is an integer greater than 3.

3. The DNA library sequence according to claim 2, wherein the position information labeling sequence consists of at least 1 N, and each N is independently A, T, C, or G; the position information labeling sequence preferably has 5 or more Ns, more preferably 5 to 50 Ns.

4. The DNA library sequence according to claim 1, wherein a first fixed sequence and a last fixed sequence of the DNA library sequence are each connected to an amplification site.

5. The DNA library sequence according to claim 1, wherein the DNA library sequence is connected to a nucleic acid molecule comprising a capture sequence capable of capturing nucleic acid in a sample.

6. The DNA library sequence according to any one of claims 1 to 5, wherein the fixed sequence contains a sequencing primer required for decoding the position information labeling sequence and a sequence that connects position information with cDNA of a subsequent single-cell transcriptome.

7. A kit, comprising:
(1) the DNA library sequence according to any one of claims 1 to 6;
(2) a reaction mix for amplification.

8. A method for preparing a chip with an increased number of probes, the method containing:
(1) synthesizing the DNA library sequence according to any one of claims 1 to 6;
(2) amplifying the DNA library sequence; the amplification is preferably bridging PCR amplification, rolling circle amplification, multiple displacement amplification, or emulsion PCR amplification;
(3) decoding the position information labeling sequence by sequencing; the sequencing is preferably ligation sequencing or sequencing by synthesis;
(4) adding a capture sequence.

9. A chip with an increased number of probes prepared by the method according to claim 8.

10. A use of the DNA library sequence according to any one of claims 1 to 6, the kit according to claim 7, or the chip according to claim 9 in a method for detecting spatial information of a nucleic acid in a sample.
